# EUROPEAN PATENT APPLICATION

(11) **EP 1 645 877 A1**
(43) Date of publication of application: **12.04.2006**
(21) Application number: 04077787.2
(22) Date of filing: 08.10.2004
(51) Int. Cl.: G01N 33/50

(54) **Method for providing a insulin resistance biomarker profile of one or more lipids**

(71) Applicant: Nederlandse Organisatie voor toegepast-natuurwetenschappelijk Onderzoek TNO, 2628 VK Delft (NL)
(72) Inventor: van der Greef, Jan, 3971 BM Driebergen (NL); Wang, Mei, 2341 BV Oegstgeest (NL); Lamers, Robert-Jan Antonius Nicolaas, 3607 WH Maarssen (NL)
(74) Representative: Winckels, Johannes Hubertus F.

(57) **Abstract**

The invention relates to a method for providing a profile of one or more lipids which is related to insulin resistance, which method comprises the steps of:
(a) determining the insulin resistance for a series of biological systems;
(b) determining the profiles of one or more lipids for a series of samples of the biological systems;
(c) determining correlations between the insulin resistance and the profiles of one or more lipids, using multivariate analysis; and
(d) developing a profile of one or more lipids based on the correlations determined in step (c).

The invention further relates to methods for providing a correlation between the insulin resistance of a biological system and a profile of one or more lipids of a sample of the biological system, and a method and device for determining insulin resistance of a biological system.

## Description

The present invention relates to a method for providing a profile of one or more lipids which is related to insulin resistance, a method for providing a correlation between a profile of one or more lipids and insulin resistance, a method for determining the insulin resistance of a biological system, and a device for determining the insulin resistance of a biological system.

Metabolic Syndrome (MSX) is characterized by insulin resistance and is closely linked to excess body fat and a sedentary lifestyle. It closely mirrors the rise in obesity in the West, and can truly be described as the disease spectrum of our times. Also known as Syndrome X or insulin resistance syndrome, the condition can lead to serious medical problems, including severe obesity, type 2 diabetes, dyslipidemia, high blood pressure, heart disease and stroke. Because the condition itself typically causes no clear symptoms in the beginning, most people are unaware they have it until it starts to attack its host from within, with devastating results.

Metabolic Syndrome has been described as "epidemic" in the US by a panel of experts from the American College of Endocrinology (ACE) in 2003 and for pre-diabetes alone the numbers are already massive and growing (20 million in US, 30 million in China). The prevalence has soared by 61% over the last decade and there is a more than 1 in 3 chance of having it after 50 years of age. For the US the estimate for Metabolic Syndrome in 2002 were 47 million Americans suffering from it (CDC, published in JAMA, 2002).

Growing attention is paid towards metabolic syndrome interventions since today most people will only be identified as having this syndrome at a stage that makes the condition almost irreversible or controllable for a longer period. For instance in the area of type 2 diabetes in 2025 the World Health Organization (WHO) estimates 300 million patients worldwide and when a person is diagnosed as type 2 diabetes typically 10-15 years have past after the start. The diagnosis is typically based on disturbances of the glucose metabolism and when elevated levels are observed several intervention strategies have been developed to slow down the process but curing or stabilizing is seldom reached.

Current Health Care systems feel the enormous pressure in the future of the fast growing population of people in a pre-diabetes state, and ideally both a more quantitative diagnostic tool and an intervention option is needed for changing the current exponential growth of the problem. Many research groups are active in this important area and criteria have been developed for a global diagnosis of MSX.
MSX is defined as the presence of three or more of the following conditions:
- Glucose intolerance ― a fasting glucose of 110 mg/dL or greater
- Blood pressure of 130/80 mm Hg or greater
- High triglycerides ― 150 mg/dL or greater
- Low levels of high-density lipoprotein (HDL) cholesterol ― less than 40 mg/dL in men and 50 mg/dL in women
- Abdominal obesity ― a waist circumference of 40 inches or more in men and 35 inches or more in women

However, the determination of insulin resistance would be a far better diagnostic tool because the used symptoms as outlined above include conditions of the later spectrum of MSX almost when clear indications such as Diabetes II, dyslipidemia, obesity, hypertension, cardiovascular disease are surfacing. Insulin resistance refers to a reduced sensitivity to the actions of insulin in the whole body or individual tissues such as skeletal muscle, myocardium, fat and liver.

For the measurement of insulin resistance an accurate method has been developed for man and animal models, the so-called euglycemic clamp methodology. This methodology measures after infusion of insulin into the body the glucose infusion rate (GIR) to bring the glucose level back to its starting level prior to the insulin infusion. The measured glucose infusion rate is a measure of the insulin resistance of the system. However, this method wherein use is made of a euglycemic clamp is expensive, complex and invasive which makes it unsuitable for any routine activity, and it is therefore limited to research purposes. In daily practise less accurate estimation methods or indices have been developed for clinical practice such as the homeostasis model assessment or HOMA-index and the Quantitative Insulin Sensitivity Check Index or QUICKI-index. The euglycemic clamp method can in special embodiments also be used to specifically measure insulin resistance of specific target tissues or cell types, which is a major advantage for understanding the underlying biological processes and consequently for developing specific intervention methods based on this knowledge.

The need to have a simple diagnostic tool or a method to design diagnostic tools for MSX in the form of measuring and predicting the insulin resistance of the body or predicting insulin resistance related to a specific target tissue is of enormous value.

Object of the present invention is to provide such a method and tool.

Surprisingly, it has now been found that the detection of lipids can be used, either alone or in combination with other biochemicals, to measure insulin resistance. Although in a late stage dysregulation of lipid metabolism is known such as for cardiovascular diseases and dyslipidemia, the relation with the early onset within metabolic syndrome has not been made. This new method and tool is possible because the lipids appear to be strongly related to the insulin resistance in an early stage of MSX. Hence, information about the lipids can directly be used to construct a diagnostic tool for metabolic syndrome.

In this respect it is important to note that it is appreciated that when a healthy state is changing towards a disease state, multiple components involved in multiple pathways will change in the biological system concerned. These changes are reflected in a so-called biomarker pattern or profile. It is further noted that in the development of a disease as in MSX, biomarker patterns change via different paths to different endpoints such as type 2 diabetes, obesity and cardiovascular disease, and for that reason the diagnostic pattern needs to be fine-tuned towards the focus of the diagnostic tool within the range of MSX and insulin resistance. Moreover the lipid profiles can be fine-tuned towards insulin resistance of a specific target tissue.

Accordingly, the present invention relates to a method for providing a profile of one or more lipids which is related to insulin resistance, which method comprises the steps of:
(a) determining the insulin resistance for a series of biological systems;
(b) determining the profiles of one or more lipids for a series of samples of the biological systems;
(c) determining the correlation between the insulin resistance and the profiles of one or more lipids, using multivariate analysis; and
(d) developing a profile of one or more lipids based on the correlation determined in step (c).

The profile of one or more lipids provided by the present invention enables the quick and effective determination of insulin resistance for a biological system. The profile is key to the development of new intervention methods and it can be used for measuring efficacy of new interventions as well as safety/toxicity related effects of the biological system or of specific parts of the biological system such as organs (e.g. liver, heart, and the like) or tissues (e.g. fat, and the like).

The invention is particularly attractive for determining insulin resistance at an early stage. In that way, proper treatments can be developed before diseases have reached an irreversible stage.

The profile of one or more lipids provides quantitative information about a particular lipid of group of lipids. The extent to which said lipid or group of lipids is present is strongly correlated to the insulin resistance within the biological system.

The biological systems can be a number of different biological systems or a number of different conditions of one particular biological system.
The insulin resistance and the profiles of one or more lipids are determined from samples obtained from a series of biological systems.

Suitable biological systems include mammals such as a human beings and animals. Preferably, the sample will be obtained from a human being for diagnostic and intervention purposes, while an animal model will be more suited for testing and designing new interventions or by studying different phenotypes or other biological relevant parameters related to MSX.

The sample preferably comprises tissue or a body fluid. Preferably, the sample comprises a body fluid. Suitable body samples include blood, urine, saliva or in special cases cerebrospinal fluid (CSF).

The profile of one or more lipids as provided in accordance with the present invention is especially useful for screening people for general health perspectives, for people with one or more symptoms of MSX, for people with a high familiar incidence of MSX or any of the related diseases, for people that have borderline values in other tests such as the impaired glucose tolerance test, fasting glucose levels, and the like.

In addition, the invention is of major importance to monitor the effect of any intervention such as life style adaptations, psychological changes, food-nutritional influences, nutraceutical, herbal medicine and medicine interventions, and the like.

It will be appreciated that for different categories of biological systems separate profiles of one or more lipids can be provided. For instance, separate profiles of one or more lipids can be provided for different sexes, age categories or different metabolic syndrome sub-types.

The lipids of which the profile is provided may suitably comprise all polar and non-polar lipids such as free fatty acids, di-and tri-glycerides, free sterols such as campesterol and β-sitosterols, cholesterol and cholesterol esters, eicosanoids, prostaglandins, steroids, isoprenoids, hopanoids, lipoperoxides, mycolic acids, ether-type lipids, phospholipids, lysophosphatidylcholines and phosphatidylcholines, sphingomyelins, phosphatidyl glycerols, phosphatidyl-inositols and phosphatidyl-serines, bile acids, ceramides and glycolipids such as glycosphingolipids and glycoglycerollipids

Preferably, the one or more lipids are chosen from the group consisting of sphingomyelins. phosphatidylcholines, lyso-phosphatidyl cholines, cholesterolesters fatty acids, di- and tri-glycerides including all homologues and isomeric forms.

The profile of one or more lipids may also include one or more other biochemicals such as genes, transcripts, proteins, metabolites and (trace) elements.

In step (a) use is made of known methods to determine the insulin resistance of biological systems. Preferably, an euglycemic clamp analysis is used to determine the insulin resistance.

In step (b) of the method according to the present invention suitably use is made of at least one spectrometric technique, at least one electromigration-based technique or at least one chromatographic technique to determine the profiles of one or more lipids.

Preferably, in step (b) use is made of the combination of liquid chromatography with mass spectrometry or gas chromatography combined with mass spectrometry or flame ionisation detection. More preferably, use is made of liquid chromatography with mass spectrometry (LCMS).

It will be appreciated that the series of biological systems will comprise at least two biological systems or at least two different conditions of a single biological system. Preferably, the number of biological systems will be in the range of from 5 to 100.

Suitable forms of multivariate analysis to be used in accordance with the present invention include, for example, principal component analysis ("PCA"), discriminant analysis ("DA"), PCA-DA, factor analysis, canonical correlation ("CC"), partial least squares ("PLS"), predictive linear discriminant analysis ("PLDA"), neural networks, multilevel/multiway/multiblock analysis, iterative target analysis, general procrustus analysis, support vector machines ("SVM"), parafac and pattern recognition techniques.

The use of the above-described multivariate analysis techniques for profiling only is well-known in the art. For a more detailed description reference can, for instance, be made to WO 03/017177 (Method and System for Profiling Biological Systems) of which the entire contents are hereby incorporated by reference.

To extract the maximum value from the data, multivariate analysis tools as outlined above may be used in concert with additional statistical and informatics strategies.

The profiles of one or more lipids can be determined in step (b) in manners known in the art. Suitably, the profiles of one or more lipids can be determined using methods and systems as described in WO 03/017177 A2, which methods and systems are hereby incorporated by reference.

The profile of one or more lipids can be obtained by including or excluding the lipoprotein fraction or the profile, for instance lipid profiles can be obtained for different fractions of the lipoproteins such as high density lipoproteins (HDL), very low density lipoproteins (VLDL) and low density lipoproteins (LDL).

The present invention also relates to a method for providing a correlation between the insulin resistance of a biological system and a profile of one or more lipids of a sample of the biological system, which method comprises the steps of:
(a) determining the insulin resistance for a series of biological systems;
(b) determining the profiles of one or more lipids for a series of samples of the biological systems;
(c) determining correlations between the insulin resistance and the profiles of one or more lipids, using multivariate analysis; and
(d) selecting a correlation between the insulin resistance and a profile of one or more lipids.

The present invention also relates to a method for determining the insulin resistance of a biological system using the profile of one or more lipids provided in accordance with the present invention, which method comprises the steps of:
(a) determining the profile of one or more lipids which is related to insulin resistance of a sample of the biological system; and
(b) determining the insulin resistance of the biological system on the basis of a correlation between the profile of the one or more lipids and the insulin resistance, which correlation is determined as described hereinbefore.

In a preferred embodiment of the method for determining the insulin resistance of a biological system, step (a) is repeated at multiple time points. This embodiment allows one to obtain an improved understanding of the dynamic component related to insulin resistance and the dynamic response of a biological system to an intervention

In another preferred embodiment of the present invention, to the profile of one or more lipids to be obtained in step (a) information is added of other biological molecules such as genes, transcripts, proteins, non-lipid metabolites and (trace) elements or the profile of one or more lipids is combined with output from other non-invasive biomarker profiling methods such as bio-imaging techniques. Suitable techniques to obtain such insulin resistance related information include non-invasive techniques such as magnetic resonance imaging, magnetic resonance spectroscopy, raman-based imaging, methods used in nuclear medicine such as Positron Emission Tomography (PET) scanning in its various forms, energetic measurements of systems such as biophoton emission, and the like. The advantage of such combined methodology enables to related the lipid profiles measured also to specific tissues and allows specific interventions to be measured and monitored. Good examples for MSX are related to imaging of the liver, muscle, fat or heart in metabolic syndrome while measuring the body fluid lipid profile or in special cases also the tissue profile.

The present invention also relates to the use of the profile of one or more lipids as provided in accordance with the present invention to determine from a sample the level of insulin resistance in a mammal. Preferably, the sample comprises a body fluid or tissue.

Since the profile of lipids enables one to determine the insulin resistance in a mammal, the profile allows one to select a proper treatment to reduce insulin resistance and/or to prevent an increase in insulin resistance.

Hence, the present invention further relates to the use of the profile of lipids as provided in accordance with the present invention for treating and/or preventing insulin resistance in a mammal.

In addition, the profile of one or more lipids as provided in accordance with the present invention can also suitably be used for designing intervention strategies in pharmaceutical, nutraceutical, functional food, herbal medicine ,nutrition applications, and the like.

The present invention also provides a device for determining insulin resistance comprising one or more detection means to determine the profile of one or more lipids as provided in accordance with the present invention of a sample of a biological system, and one or more means to determine the insulin resistance on the basis of a pre-established correlation between insulin resistance and said profile.

The device in accordance with the present invention can attractively be used to select a composition capable of treating and/or preventing insulin resistance. Suitable compositions include pharmaceutical compositions, food, functional food, nutraceutical or herbal products, chemical mixtures, natural products or extracts thereof, and the like.

The attractiveness to use lipids for measuring the insulin resistance is illustrated by means of steps (a)-(c) in an experiment using the APOE3 Leiden transgenic mouse model which has been used for the induction of insulin resistance using a special diet regime.

APOE*3Leiden (E3L) transgenic mice exhibit elevated plasma cholesterol and triglyceride levels, mainly confined to the VLDL/LDL lipoprotein fraction. Extensive previous research showed that, in contrast to wild-type mice, E3L mice are highly responsive to sucrose, fat and cholesterol feeding as far as the effects on plasma VLDL and chylomicron levels are concerned. In addition, E3L mice are also sensitive to high fat-induced insulin resistance (e.g. Muurling et al., 2002, metabolism 51:695-701). The male mice are used for metabolic syndrome and as an early onset diabetes type II model based on the insulin resistance developed. Male heterozygous E3L mice are from a SPF breeding stock, and housed during the experiment in clean-conventional animal rooms (relative humidity 50-60%, temperature ~21°C, light cycle 6 am to 6 pm). Mice are supplied with food and acidified tap water ad lib. The effects on insulin sensitivity in high fat and high calorie (HFHC) diet-induced insulin resistance APOE*3 Leiden mouse are measured using euglycemic clamp analysis at the last time point.

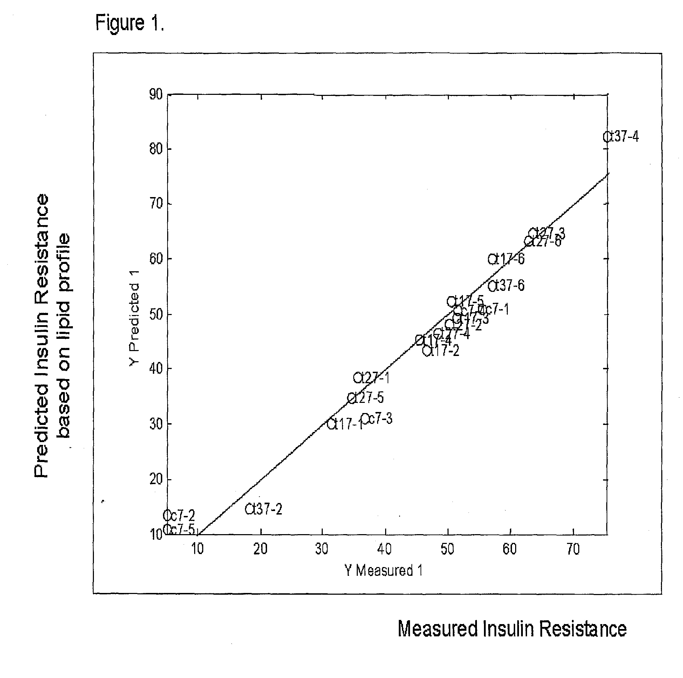

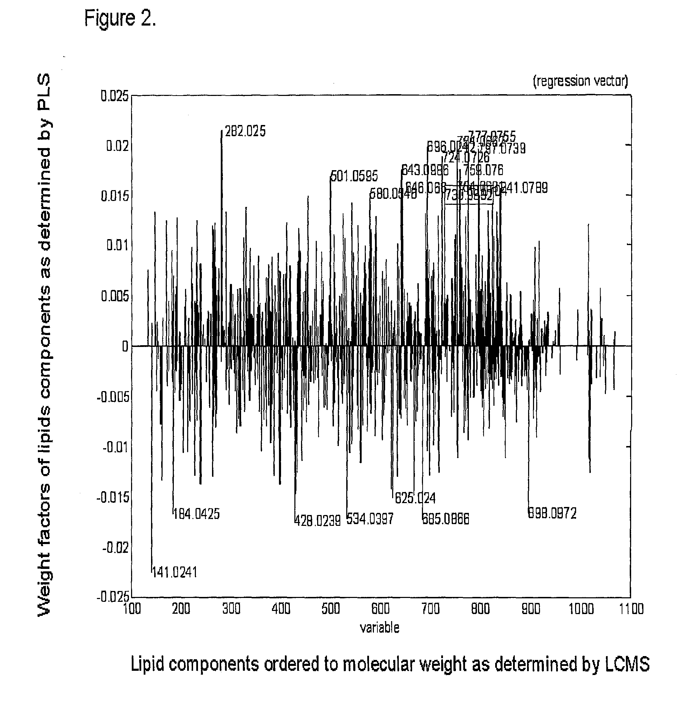

In step (a) plasma was selected from APOE*3 Leiden mice after induction of insulin resistance using a high fat and high calorie (HFHC) diet, the insulin resistance was measured by euglycemic clamp analysis for each individual mouse. In step (b) a lipid profiling method was used to profile the class of lipids in the most extensive way with high coverage of this biological class of components. This profiling can be done with various analytical techniques but preferably with gas chromatography using mass spectrometry or flame ionisation detection or by liquid chromatography coupled to mass spectrometry (LC/MS) or by any combination of the mentioned methods or by any other method capable of profiling and quantification of lipids. In this example LC/MS profiling was chosen. In step (c) a multivariate regression method was selected, partial-least-squares (PLS) after orthogonal signal correction (OSC) to determine which components can best be selected to predict the insulin resistance. The outcome is given in Figure 1 showing the correlation between predicted insulin resistance values by the lipid method and the observed insulin resistance as measured via the euglycemic clamp analysis. The correlation is very high demonstrating the high information content of the lipid molecules in respect to insulin resistance. A more in depth interpretation of the individual components can be obtained by examining the components with the highest weight factor in PLS, see Figure 2.

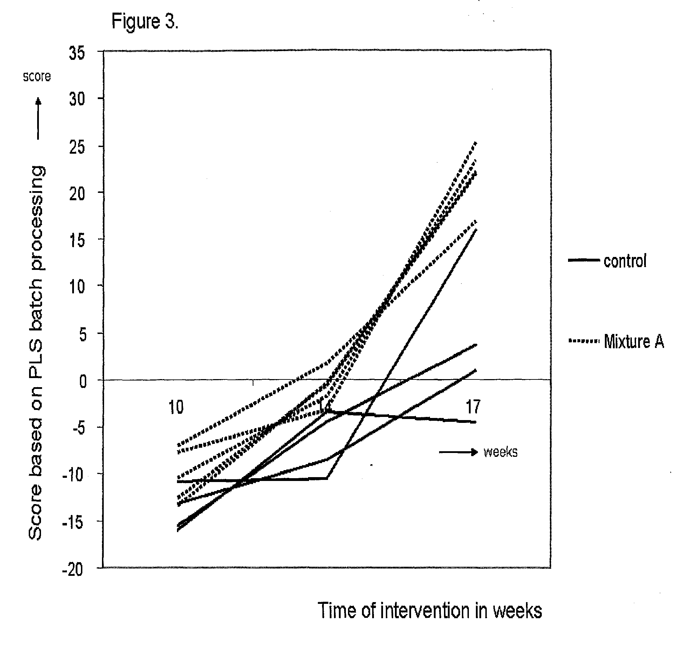

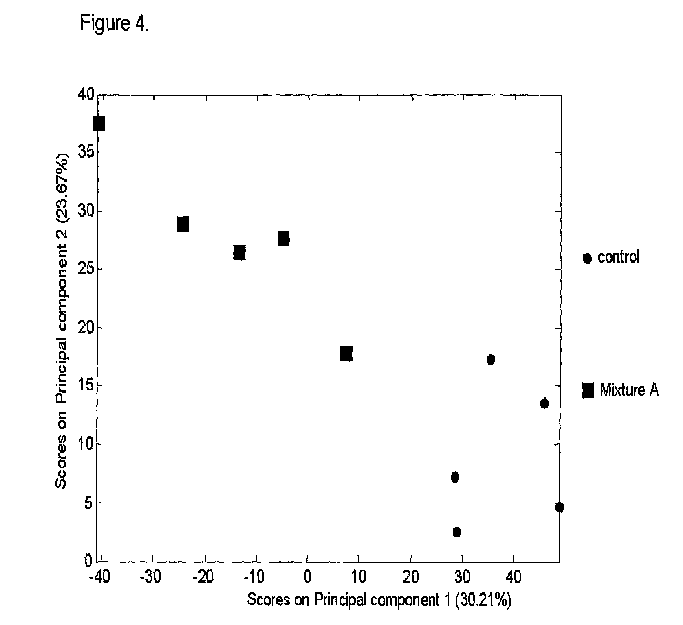

A further embodiment of the present invention includes the use of longitudinal and multi-level information based on lipids or on lipids combined with other biochemical components for measuring insulin resistance. The underlying invention is based on the fact that dysregulation of systems in an early state is reflected in the dynamics of that system, a good example being the glucose tolerance test which in fact measures the dynamic response of the system to a challenge with glucose. The oral glucose tolerance test is typically conducted by measuring blood glucose levels five times over a period of 3 hours. It is basically the measurement of the body's ability to appropriately handle the excess sugar presented after drinking a high glucose drink. Such longitudinal, time-resolved or temporal data reflecting a dysregulation or change in homeostasis of a system can be obtained by measurement of the lipid profile at various time points with or without perturbation of the system. The frequency of the sampling can depend on the application, for instance could be in the seconds to minutes-hours time frame with or without perturbing the systems with chemicals, nutrition or special variants thereof, herbal medicine, drugs etc. Longer sampling times can be of importance for slower interventions and can extend from hours to days and months or even longer. The detection of specific longitudinal lipid profiles for prediction of insulin resistance can be detected but not limited to regression methods such as ridge regression, principal component regression, PLS-batch processing for more continuous data or for example for discontinuous data by linear discriminant analysis (LDA), principle component analysis combined with discriminant analysis (PCDA), PLS-DA, support vector machines (SVM), neural networks etc. Such longitudinal profiles can be excellent for evaluation of the efficacy and dose-response effect of interventions. An example is given in Figure 3 for a herbal medicine intervention, which is one of the most complicated examples, in an experiment using the APOE3 Leiden transgenic mouse model as described above using a herbal medicine intervention to reveal the dose-response profile based on the lipid profile indicative for insulin resistance. In this particular case PLS-batch processing was used to reveal the time effects of the intervention, which can be related to insulin resistance. Moreover when principal component analysis is performed on the PLS-scores, see Figure 4, the treatment effect is clearly visible and interpretable at the lipid level.

## Claims

1. A method for providing a profile of one or more lipids which is related to insulin resistance, which method comprises the steps of:
(a) determining the insulin resistance for a series of biological systems;
(b) determining the profiles of one or more lipids for a series of samples of the biological systems;
(c) determining correlations between the insulin resistance and the profiles of one or more lipids, using multivariate analysis; and
(d) developing a profile of one or more lipids based on the correlations determined in step (c).

2. A method according to claim 1, wherein the sample is obtained from a mammal.

3. A method according to claim 1 or 2, wherein the sample comprises tissue or a body fluid.

4. A method according to claim 3, wherein the sample comprises a blood sample, a urine sample, a saliva sample or a cerebrospinal fluid sample.

5. A method according to any one of claims 1-4, wherein in step (a) the insulin resistance is determined using an euglycemic clamp.

6. A method according to any one of claims 1-5, wherein in step (b) use is made of at least one spectrometric technique, at least one electromigration-based technique or at least one chromatographic technique to determine the profiles of lipids.

7. A method for providing a correlation between the insulin resistance of a biological system and a profile of one or more lipids of a sample of the biological system, which method comprises the steps of:
(a) determining the insulin resistance for a series of biological systems;
(b) determining the profiles of one or more lipids for a series of samples of the biological systems;
(c) determining correlations between the insulin resistance and the profiles of one or more lipids, using multivariate analysis; and
(d) selecting a correlation between the insulin resistance and a profile of one or more lipids.

8. A method for determining the insulin resistance of a biological system using a profile as defined in any one of claims 1-7, which method comprises the steps of:
(a) determining the profile of one or more lipids of a sample of the biological system; and
(b) determining the insulin resistance of the biological system on the basis of a correlation between the profile of one or more lipids and insulin resistance, which correlation is determined as described in any one of claims 1-7.

9. A method according to claim 8, wherein in step (a) use is made of at least one spectrometric technique, at least one electromigration-based technique or at least one chromatographic technique to determine the profile of lipids.

10. A method according to claim 8 or 9, wherein to the profile of the one or more lipids information is added of other biological molecules such as genes, transcripts, proteins, non-lipid metabolites and (trace) elements or the profile of one or more lipids is combined with other non-invasive biomarker profiles or patterns.

11. A method according to any one of claims 8-10, wherein step (a) is repeated at multiple time points.

12. Use of the profile of one or more lipids as defined in any one of claims 1-7 to determine the level of insulin resistance in a biological system.

13. Use of the profile of one or more lipids as defined in any one of claims 1-7 for treating and/or preventing insulin resistance in a biological system.

14. Use of the profile of the one or more lipids as defined in any one of claims 1-7 for designing intervention strategies in pharmaceutical, nutraceutical, functional food, herbal medicine and nutrition applications.

15. A device for determining the insulin resistance of a biological system comprising one or more detection means to determine the profile of one or more lipids as defined in any one of claims 1-7 of a sample of the biological system, and one or more means to determine the insulin resistance on the basis of a correlation between insulin resistance and the profile of one or more lipids.

16. Use of a device according to claim 15 for selecting a composition capable of treating and/or preventing insulin resistance.
